# EUROPÄISCHE PATENTSCHRIFT

(11) **EP 0 639 077 B1**
(45) Veröffentlichungstag und Bekanntmachung des Hinweises auf die Patenterteilung: **26.03.1997**
(21) Anmeldenummer: 93911730.5
(22) Anmeldetag: 30.04.1993
(51) Int. Cl.: A61K 31/56, A61K 31/565

(54) **ARZNEIMITTEL ZUR ERHÖHUNG DES TESTOSTERONSPIEGELS**
DRUG FOR INCREASING THE LEVEL OF TESTOSTERONE IN THE BODY
MEDICAMENT ELEVANT LE TAUX DE TESTOSTERONE

(30) Priorität: 06.05.1992 DE 4214953
(43) Veröffentlichungstag der Anmeldung: 22.02.1995
(73) Patentinhaber: ARROWDEAN LTD., IE-Dublin 2 (IE)
(72) Erfinder: MATTERN, Claudia, D-8130 Starnberg (DE); HÄCKER, Rüdiger, A-6600 Greiz (AT)
(74) Vertreter: Winkler, Andreas, Dr.
(86) Internationale Anmeldenummer: DE9300397
(87) Internationale Veröffentlichungsnummer: WO9321924

(56) Entgegenhaltungen:
- EP-A- 0 349 091
- US-A- 3 284 303
- US-A- 4 877 774
- US-A- 5 053 403
- Z. HAUTKR., Bd. 61, Nr. 19, 1986, Seiten 1372-1376; GABRIELA STOBBE ET AL.: 'Die perkutane Resorption von Testosteron, Androstendion und Oestradiol und ihre Wirkung auf die Steroidserumspiegel'

## Beschreibung

Die Erfindung betrifft ein Arzneimittel zur Erhöhung des Testosteronspiegels beim Menschen.

Die Hauptwirkung des Steroidhormons Testosteron besteht in der Ausprägung der primären und sekundären Geschlechtsmerkmale des Mannes sowie der Erhaltung der damit verbundenen Funktionen. Neben dieser Hauptwirkung entfaltet das Testosteron eine Reihe von Seitenwirkungen, die für die Belastbarkeit und Leistungsfähigkeit des menschlichen Organismus von wesentlicher Bedeutung sind. Hierzu gehören die Aufrechterhaltung einer anabolen Stoffwechselsituation, die Wiederherstellung der Leistungsfähigkeit des Menschen nach erschöpfenden Belastungen und die Erhöhung der psychophysiologischen Belastbarkeit und Streßresistenz.

Die Wirkungsmechanismen von Testosteron sind ausführlich untersucht worden. Die Seitenwirkungen auf den psychophysiologischen Zustand werden nach neuesten Untersuchungen auf das Vorhandensein von Testosteronrezeptoren im zentralen Nervensystem zurückgeführt.

Testosteron ist im Blut zu über 90% an Eiweiß gebunden; die biologisch wirksame Komponente ist das freie Testosteron, das 4-8% der Gesamtkonzentration im Blut ausmacht. Die Testosteronkonzentration im Blut unterliegt einem physiologischen Tagesgang (höchste Konzentration morgens), einem jahreszeitlichen Gang (niedrigste Konzentration im Mai) und Beeinflussungen durch Lebensumstände und Alterungsprozesse.

Die Gesamttestosteronkonzentration im Blut ist unter Normalbedingungen individuell sehr stabil. Hohe körperliche Belastungen, lang andauernde Streßsituationen und ungünstige Ernährungsregimes erniedrigen aber den Blutspiegel. Mit zunehmendem Alter tritt, besonders deutlich beim Mann ab etwa 35 eine Verringerung der Konzentration an freiem Testosteron ein. Diese Veränderungen führen zu einer verminderten allgemeinen Leistungsfähigkeit, zu einem höheren Zeitbedarf für die Wiederherstellung des Organismus nach erschöpfenden Belastungen und zu einer Reduzierung der psychophysiologischen Belastbarkeit sowie der Streßresistenz. Untersuchungen an physisch und psychisch hoch belasteten Personen haben ergeben, daß eine Anhebung des Testosteronspiegels in den oberen Bereich der individuellen physiologischen Schwankungsbreite zu einer Aufhebung dieser negativen Situation und zu einer Erhöhung der allgemeinen Leistungsfähigkeit führt. Ein Anstieg der Konzentration über die individuelle obere Normgrenze zeigt dagegen keinen besseren therapeutischen Effekt, sondern ruft Nebenwirkungen hervor.

Die Erhöhung des Testosteronspiegels beim Menschen im Sinne einer Substitution ist daher zum Bestandteil von präventiven und therapeutischen Konzepten in der Altersmedizin, insbesondere des Mannes, geworden. Die Zufuhr erfolgt üblicherweise peroral oder in öliger Lösung intramuskulär, teilweise als Depotpräparat.

Mit diesen Darreichungsformen verbinden sich folgende Nachteile:
- die Beeinflussung des Blutspiegels ist insgesamt schlecht steuerbar;
- die individuelle Ausgangssituation und Belastung können für die Medikation nicht ausreichend berücksichtigt werden;
- die perorale und intramuskuläre Zufuhr ziehen eine Verstoffwechslung über den Kreislauf Leber-Galle-Darm-Leber nach sich ("first-pass-Effekt");
- dieser Effekt verringert die Bioverfügbarkeit und erfordert die Zufuhr höherer Dosen mit resultierender höherer Belastung des Stoffwechsels;
- die Zufuhr höherer Dosen kann zu unerwünschten Anstiegen in der Gesamtkonzentration führen, die über den physiologischen Regelmechanismus die körpereigene Testosteronproduktion reduzieren.

Der vorliegenden Erfindung liegt daher die Aufgabe zugrunde, ein Arzneimittel zur Erhöhung des Testosteronspiegels beim Menschen zur Verfügung zu stellen, dessen Applikation der intramuskulären Zufuhr von Testosteron in seiner Wirkung gleichwertig ist, die oben angegebenen Nachteile vermeidet mit einer wesentlich geringeren Dosis auskommt und eine Betonung der Seitenwirkung auf das zentrale Nervensystem ermöglicht.

Erfindungsgemäß wird diese Aufgabe durch die Verwendung mindestens einer der Verbindungen Androstendion, Progesteron oder 17-α-Hydroxyprogesteron zur Herstellung eines Arzneimittels in einer pernasal applizierbaren Darreichungsform Zur Erhöhung des Testosteronspiegels beim Menschen gelöst.

Eine besonders vorteilhafte Ausführungsform der Erfindung ist gekennzeichnet durch einen bevorzugten Gehalt von 3,5 bis 15 mg wirksamer Substanz pro Pumpstoß.

Es konnte gezeigt werden, daß durch den Einsatz eines Vorläufers (Präcursors) von Testosteron, der erst im Organismus in die wirksame Substanz umgewandelt wird, eine komplexere Reaktion des Steroidstoffwechsels entsteht, die insgesamt ausgewogener ist und den physiologischen Bedingungen besser entspricht, so daß insgesamt eine optimale Wirkung unter Vermeidung von Nebenwirkungen erzielt werden kann.

Tierversuche am Meerschweinchen haben die schnelle Umwandlung von radioaktiv markiertem Androstendion, Progesteron oder 17-α-Hydroxyprogesteron in Testosteron grundsätzlich nachgewiesen.

Beim Menschen werden 50 bis 100 mg peroral zugeführtes Androstendion Progesteron oder 17-α-Hydroxyprogesteron ebenfalls schnell in Testosteron umgewandelt. Bei Zufuhr von Androstendion tritt z.B. bereits nach 15 Minuten im Blut eine Erhöhung der Gesamtkonzentration an Testosteron von 40 bis 83% (50 mg) bzw. 111 bis 237% (100 mg) auf. Es kommt zu einer Erhöhung des Anteils an freiem (biologisch wirksamem) Testosteron, wobei das Auftreten des Konzentrationsmaximums und der Verlauf des Blutspiegels bei positiver Grundreaktion deutliche, sich wiederholende individuelle Unterschiede zeigen.

Im Falle der erfindungsgemäßen pernasalen Applikation mittels Dosierspray konnten bei einmaliger Zufuhr von 3,5 bis 15 mg Androstendion, Progesteron oder 17-α-Hydroxyprogesteron Anhebungen des Testosteronspiegels im Blut um 34 bis 97% erreicht werden. Diese sind in Ausmaß und Zeitablauf vergleichbar mit den Ergebnissen, die bei peroraler Zufuhr wesentlich höherer Dosen oder intramuskulärer Zufuhr von Testosteronpropionat erzielt werden konnten. Im Gegensatz zur peroralen und intramuskulären Verabreichung findet bei der pernasalen Applikation keine wesentliche "first-pass"-Metaboliserung des Vorläufermoleküls statt.

Daraus resultiert die gute Steuerbarket der Beeinflussung, die durch mehrfache Applikation nachgewiesen werden konnte. Die individuelle Reaktionslage wird durch die Regulationsmechanismen des Stoffwechsels berücksichtigt. Es wird eine angepaßte Erhöhung des freien Testosterons erzielt die in Ausmaß und Kinetik mit den Werten vergleichbar ist, die bei percraler Applikation der zehnfach höheren Dosis erhalten werden.

Bei mehrfacher pernasaler Applikation wurde ein deutlicher Langzeiteffekt gefunden. Drei bis vier Tage nach der letzten Applikation tritt eine erneute Erhöhung des Testosteronspiegels um 48 bis 97% im Blut auf, die für weitere sechs bis sieben Tage bestehen bleibt. Diese Reaktion ist wahrscheinlich auf eine Beeinflussung des Regelkreises für die körpereigene Testosteronproduktion zurückzuführen.

Die pernasale Applikation erleichtert darüberhinaus den Übertritt in den Liquor cerebrospinalis sowie in andere Gewebe und Organe des menschlichen Organismus. Da die Überwindung der Blut-Hirn-Schranke für alle zentralnerval wirksamen Pharmaka ein großes Problem darstellt, ist insbesondere der erleichterte Zugang zum Liquor cerebrospinalis über die pernasale Applikation ein besonderer Vorteil des erfindungsgemäßen Arzneimittels, da hierdurch erstmals die Beeinflussung der Testosteron- Rezeptoren im Gehirn ermöglicht wird, was einen neuen therapeutischen Ansatz für Testosteron darstellt. Durch diese Beeinflussung des zentralen Nervensystems wird vermutlich auch die weiter unten beschriebene Verbesserung der psychophysiologischen Leistungsfähigkeit erreicht.

Im Metabolitprofil des Urins tritt eine Erhöhung des Quotienten Testosteron/Epitestosteron auf. Sie ist bei pernasaler Applikation jedoch nicht so markant (3,8 bis maximal 14,3) und normalisiert sich am auf die Zufuhr folgenden Tage, wobei der Testosteronspiegel im Blut noch angehoben bleibt.

Der Einsatz des Nasensprays über 6 Tage (Tagesdosis 5 bis 7 mg) bei physisch und psychisch hoch belasteten Personen im mittleren Lebensalter zeigt eine Verkürzung der Regeneration nach erschöpfenden Belastungen und eine ausgeglichenere Stoffwechselsituation. Besonders hervorzuheben ist, daß alle Probanden unaufgefordert über eine höhere psychophysiologische Belastbarkeit und eine verbesserte Streßresistenz berichteten.

Die in der vorstehenden Beschreibung, sowie in den Ansprüchen offenbarten Merkmale der Erfindung können sowohl einzeln als auch in beliebiger Kombination für die Verwirklichung der Erfindung in ihren verschiedenen Ausführungsformen wesentlich sein.

## Patentansprüche

1. Verwendung mindestens einer der Verbindungen Androstendion, Progesteron oder 17-α-Hydroxyprogesteron zur Herstellung eines Arzneimittels in einer pernasal applizierbaren Darreichungsform zur Erhöhung des Testosteronspiegels beim Menschen.

## Claims

1. Use of at least one of the following compounds: androstenedione, progesterone or 17-α-hydroxyprogesterone for producing a drug in a form of administration for nasal application, for increasing the testosterone level in man.

## Revendications

1. Utilisation d'au moins l'une des compositions que sont l'androstendione, la progestérone ou la 17-α-hydroxyprogestérone, pour la préparation d'un médicament dans une forme d'administration à possibilité d'application pernasale, pour l'élévation du niveau de testostérone chez l'homme.
